# FASCICULE DE BREVET EUROPEEN

(11) **EP 2 293 835 B1**
(45) Date de publication et mention de la délivrance du brevet: **30.10.2013**
(21) Numéro de dépôt: 09766030.2
(22) Date de dépôt: 12.05.2009
(51) Int. Cl.: A61M 16/01, A61M 16/10, A61M 16/12, G01N 27/18, G01N 33/00, G01N 33/497

(54) **AMELIORATION DE LA PRECISION DE MESURE DE LA TENEUR EN XENON DANS UN APPAREIL D'ANESTHESIE VENTILATOIRE**
ERHÖHUNG DER GENAUIGKEIT VON XENONANTEILSMESSUNGEN IN EINER ANÄSTHESIEBEATMUNGSVORRICHTUNG
IMPROVEMENT IN THE PRECISION OF XENON CONTENT MEASUREMENT IN A VENTILATORY ANAESTHESIA APPARATUS

(30) Priorité: 27.05.2008 FR 0853430
(43) Date de publication de la demande: 16.03.2011
(73) Titulaire: L'Air Liquide Société Anonyme pour l'Etude et l'Exploitation des Procédés Georges Claude, 75007 Paris (FR)
(72) Inventeur: BLANDIN, Richard, F-75020 Paris (FR); CARIOU, Bernard, F-78180 Montigny Le Bretonneux (FR); DAVIET, Christian, F-75011 Paris (FR); DUSSUD, Sophie, 92500 Rueil Malmaison (FR); KISSI, Noureddine, F-92360 Meudon La Foret (FR)
(74) Mandataire: Pittis, Olivier
(86) Numéro de dépôt international: PCT/FR2009/050859
(87) Numéro de publication internationale: WO 2009/153468

(56) Documents cités:
- WO-A-2004/024053
- WO-A-2007/068849
- DE-A1- 3 742 880
- DE-U1- 29 613 243
- FR-A- 2 862 227

## Description

La présente invention porte sur un appareil d'anesthésie ventilatoire, par administration de xénon gazeux aux voies aériennes d'un patient, muni d'un dispositif de mesure de la concentration de xénon permettant d'obtenir une bonne précision de mesure.

On connaît de nombreux appareils d'anesthésie ventilatoire pouvant être utilisés pour réaliser l'anesthésie d'un patient devant subir une intervention chirurgicale ou analogue, en lui administrant par inhalation un mélange gazeux anesthésique classique composé de N₂O, d'agents halogénés, par exemple du sevoflurane, isoflurane, desflurane... A ce titre, on peut se reporter aux documents EP-A-983771 et EP-A-1120126.

Le xénon est un gaz anesthésique connu depuis le début des années 1950 et qui est de plus en plus utilisé en milieu médical, notamment car il est particulièrement adapté à l'anesthésie des patients fragiles (patients âgés, opérations, longues, chirurgie cardiaque, neurochirurgie...), du fait notamment d'une incidence quasi-nulle sur la pression sanguine durant l'anesthésie et d'une quasi-absence d'effets secondaires et de nocivité.

Toutefois, une anesthésie réalisée avec du xénon nécessite un suivi ou monitorage des concentrations de xénon dans le flux gazeux administré au patient, c'est-à-dire requiert de pouvoir déterminer en temps réel la concentration en xénon dans le flux anesthésique. A ce titre, on peut se reporter par exemple aux documents EP-A-1499377, EP-A-1318797 ou EP-A-523315.

Pour mesurer la concentration de xénon dans un tel mélange gazeux, il est classique d'utiliser un spectromètre de masse ou un chromatographe. Or, ces techniques présentent des inconvénients de coût et surtout de difficulté de mise en oeuvre car leur intégration dans les appareils d'anesthésie existants nécessite des efforts de développement et d'adaptation importants.

Une alternative a été proposée par le document WO-A-2007/068849 qui enseigne un appareil d'anesthésie ventilatoire d'un patient par administration d'un gaz contenant du xénon gazeux comprenant des moyens de détermination de concentration de xénon pour déterminer la teneur en xénon gazeux dans le circuit principal de gaz en circuit ouvert ou fermé.

Dans cet appareil, un ou plusieurs capteurs à fils chauds comportant chacun au moins un fil en matériau conducteur de l'électricité, de préférence en métal, est en contact direct avec le flux gazeux contenant le xénon, et des moyens de calcul coopérant avec le ou les capteurs à fils chauds de manière à déterminer la concentration de xénon dans ledit flux gazeux à partir d'une mesure de tension effectuée par les moyens de mesure de tension aux bornes d'au moins un fil chaud ou d'une résistance placée en série avec au moins un fil chaud, lorsque ledit au moins un fil chaud est en contact avec le flux gazeux et est parcouru par un courant électrique.

Si cet appareil permet de déterminer avec une précision suffisante la concentration en xénon délivrée au patient pendant une anesthésie gazeuse de manière à garantir une efficacité d'anesthésie et une sécurité accrue pour le patient, tout en étant d'architecture simple et de coût modique, il est apparu en pratique que, dans certains cas, notamment en cas de début d'occlusion pouvant apparaître au fur et à mesure de l'utilisation par accumulation d'humidité dans la ligne de prélèvement ou en cas de vieillissement normal ou prématuré de la pompe d'aspiration, la stabilité du ou des signaux mesurés peu être dégradée par les fluctuations du débit de la pompe de prélèvement des échantillons.

En effet, lorsque l'échantillon de gaz à mesurer est susceptible de variation de débit ou d'oscillations fluidiques dues par exemple à la pompe de prélèvement de l'échantillon gazeux, cela se traduit, dans une certaine mesure, par des perturbations au niveau de la mesure de la concentration en xénon.

Bien que ces perturbations conduisent à une mesure de concentration qui reste très acceptable, il est souhaitable de pouvoir supprimer ces perturbations et d'éviter ces fluctuations de mesure de teneur en xénon.

En d'autres termes, le problème à résoudre est d'améliorer l'appareil décrit par le document WO-A-2007/068849 de manière à supprimer toutes les perturbations de mesure de la concentration en xénon dans le flux gazeux et donc renforcer la stabilité de mesure, c'est à dire apporter à ce dispositif une plus grande précision de mesure, de manière à permettre d'obtenir un suivi ou monitorage encore plus efficace, fiable et précis des concentrations de xénon gazeux dans un mélange gazeux d'anesthésie à base de xénon contenant, en outre, en quantité variable, c'est-à-dire de 0 à 100% en volume, de l'un ou plusieurs des composés principaux suivants : oxygène (O₂), azote (N₂), protoxyde d'azote (N₂O), dioxyde de carbone (CO₂), composés halogénés de type isoflurane, enflurane, desflurane, sevoflurane ou halothane, éthanol, et éventuellement des traces ou quantités faibles (<1 %) de l'un ou plusieurs des composés mineurs suivants : acétone, méthane, monoxyde de carbone (CO), argon, hélium...

A cet effet, l'invention propose un appareil d'anesthésie ventilatoire d'un patient par administration d'un gaz contenant du xénon gazeux comprenant :
- un circuit principal de gaz en circuit ouvert ou fermé comportant une branche inspiratoire pour acheminer un mélange gazeux contenant du xénon vers le patient et une branche expiratoire pour véhiculer le mélange gazeux contenant du xénon expiré par le patient, et
- des moyens de détermination de concentration de xénon conçus pour et aptes à déterminer la teneur en xénon gazeux dans au moins une partie du circuit principal, lesdits moyens de détermination de concentration de xénon comprenant au moins un capteur à fil(s) chaud(s) comportant au moins un fil conducteur de l'électricité en contact direct avec au moins une partie du flux gazeux contenant le xénon,
   caractérisé en ce que :
- au moins un capteur à fil(s) chaud(s) comportant au moins un fil conducteur est agencé sur une ligne principale d'acheminement de gaz comportant une ligne de dérivation se raccordant fluidiquement à ladite ligne principale d'acheminement de gaz en amont et en aval dudit au moins un fil conducteur, en considérant le sens de circulation du gaz dans la ligne principale, et
- au moins une première électrovanne est agencée au niveau du raccordement amont de la ligne de dérivation à ladite ligne principale de manière à diriger le flux gazeux contenant le xénon soit vers la ligne principale sur laquelle est agencé ledit au moins un fil conducteur, soit vers la ligne de dérivation.

Selon le cas, l'appareil de l'invention peut comprendre l'une ou plusieurs des caractéristiques suivantes :
- la ligne principale d'acheminement de gaz et la ligne de dérivation sont des ramifications d'une ligne de dérivation communiquant fluidiquement avec le circuit principal et au moins une première électrovanne est agencée à l'intersection de ladite la ligne de dérivation, de la ligne principale d'acheminement de gaz et la ligne de dérivation, de préférence le raccordement de la ligne de dérivation au circuit principal se fait sur la branche inspiratoire et/ou sur la branche expiratoire et/ou en un site localisé à proximité immédiatement de la bouche du patient, de préférence encore au niveau d'un site de raccordement entre la branche inspiratoire et la branche expiratoire dudit circuit principal, par exemple au niveau d'une pièce de raccordement en Y ou d'un filtre bactériologique agencé sur le circuit principal.
- au moins un capteur à fil(s) chaud(s) est agencé directement sur la branche inspiratoire ou expiratoire du circuit principal, et en ce que la ligne principale d'acheminement de gaz et la ligne de dérivation sont des ramifications de la branche inspiratoire ou expiratoire du circuit principal, et au moins une première électrovanne est agencée à l'intersection de la branche inspiratoire ou expiratoire, de la ligne principale et la ligne de dérivation.
- au moins une deuxième électrovanne est agencée au niveau du raccordement aval de la ligne de dérivation à ladite ligne principale.
- au moins un clapet anti-retour est agencé sur la ligne principale d'acheminement de gaz, entre au moins un fil conducteur et le raccordement aval de la ligne de dérivation à ladite ligne principale.
- au moins un deuxième capteur à fil conducteur est agencé sur la ligne de dérivation qui mesure la concentration en xénon par exemple pendant la phase expiratoire alors qu'au moins un fil conducteur mesure la concentration pendant la phase inspiratoire ou inversement.
- au moins un capteur à fil(s) chaud(s) est agencé sur la branche inspiratoire, en amont ou en aval du capteur de débit inspiratoire pour permettre de mesurer la fraction inspirée de xénon.
- au moins un capteur à fil(s) chaud(s) est agencé sur la branche expiratoire, en amont ou en aval du capteur de débit expiratoire pour permettre de mesurer la fraction expirée de xénon.
- les électrovannes sont commandées par des moyens de pilotage ou par une interface indépendante.
- il comprend des moyens d'alimentation en xénon gazeux reliés au circuit principal pour alimenter la branche inspiratoire du circuit principal avec un gaz contenant du xénon, et des moyens de calcul coopérant avec au moins un capteur à fil(s) chaud(s) de manière à déterminer la concentration de xénon dans ledit flux gazeux, des moyens de génération de courant électrique apte à et conçus pour générer un courant un électrique dans au moins un fil chaud dudit au moins un capteur à fil(s) chaud(s), des moyens de mesure de tension aptes à mesurer au moins une valeur de tension aux bornes d'au moins un fil chaud dudit au moins un capteur à fil chaud ou aux bornes d'au moins une résistance agencée en série avec au moins un fil chaud dudit au moins un capteur à fil chaud, lorsque ledit au moins un fil chaud est en contact avec le flux gazeux et est parcouru par un courant électrique d'intensité non nulle, et les moyens de calcul coopèrent avec les moyens de mesure de tension de manière à déterminer, à partir de la mesure de tension effectuée par lesdits moyens de mesure de tension, la concentration de xénon dans ledit flux.

Le fonctionnement de l'appareil de la présente invention est donc basé sur une utilisation d'un ou plusieurs capteur(s) à fil(s) chaud(s) pour déterminer, en temps réel, la concentration instantanée et/ou moyenne de xénon présente dans le gaz d'anesthésie en phase inspiratoire et/ou en phase expiratoire. L'invention permet également de fournir la concentration en xénon gaz des inspirés et/ou expirés.

Le principe de mesure du débit d'un gaz anesthésique au moyen d'un (ou plusieurs) capteur(s) à fil(s) chaud(s) est donné dans le document WO-A-2007/068849 auquel on se reportera pour plus de détail, notamment sur la manière de calculer la concentration en xénon à partir de la (ou des) valeur de tension mesurée aux bornes du (ou des) fil chaud ou d'une résistance placée en série avec au moins un fil chaud, lorsque le fil chaud considéré est en contact avec le flux gazeux contenant le xénon et est parcouru par un courant électrique d'intensité non nulle.

L'invention va être mieux comprise grâce à la description suivante faite en références aux figures annexées, parmi lesquelles :
- la figure 1 représente un premier mode de réalisation d'un appareil selon l'invention, et les figures 2 et 3 représentent des variantes du mode de réalisation de l'appareil de la figure 1,
- la figure 4 représente un second mode de réalisation d'un appareil selon l'invention, et
- les Figures 5 à 8 sont des monogrammes de débit gazeux.

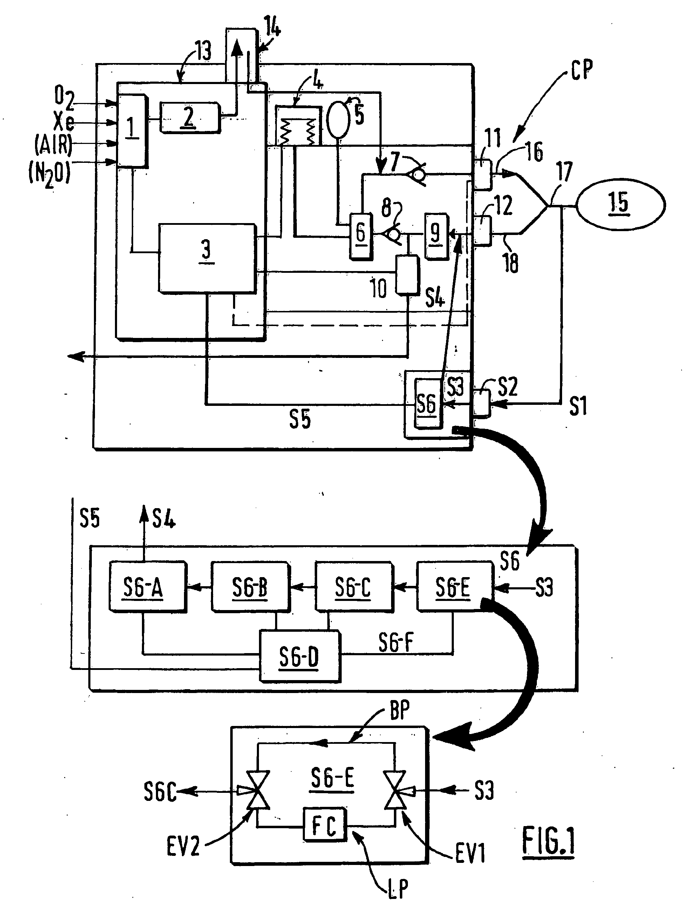

L'appareil ou ventilateur des Figures 1 à 4 comprend un bloc d'entrée 1 comprenant des moyens de raccordement auxquels viennent se raccorder la source de xénon et les autres sources de gaz alimentant l'appareil d'anesthésie, telles des bouteilles de gaz ou un réseau mural, en particulier pour les sources d'air (AIR), d'oxygène (O₂) et/ou de protoxyde d'azote (N₂O).

Ce bloc 1 est en communication fluidique avec l'entrée d'un mélangeur 2 où se fait le mélange du xénon avec le ou les autres gaz qui sont destinés à former le mélange gazeux anesthésique, en particulier de l'oxygène en une quantité suffisante pour le patient (non hypoxique), et la sortie du mélangeur 2 alimente en mélange gazeux, une cuve à halogénées 14, montée sur un support 13 de cuve, contenant un composé halogéné destiné à être entraîné par le flux de gaz anesthésique jusqu'au patient 15.

Le mélange gazeux halogéné sortant de la cuve 14 est introduit dans un circuit principal CP ou circuit patient comportant une branche inspiratoire 16 pour acheminer le mélange gazeux vers le patient 15 et une branche expiratoire 18 pour récupérer tout ou partie du gaz expiré (chargé en CO₂) par le patient 15. Les branches inspiratoire 16 et expiratoire 18 forment un circuit en boucle ou circuit fermé. La liaison entre les branches inspiratoire 16 et expiratoire 18 avec le patient 15 se fait via, par exemple, une pièce 17 en Y et un masque respiratoire, une sonde trachéale ou analogue.

Des clapets anti-retour inspiratoire 7 et expiratoire 8 sont de préférence aménagés respectivement sur lesdites branches inspiratoire 16 et expiratoire 18. La branche expiratoire 18 comporte un dispositif 9 absorbeur de CO₂ comprenant une cuve remplie d'un matériau absorbant, telle de la chaux, permettant d'éliminer le CO₂ expiré par le patient 15 et véhiculé par le gaz expiré dans la branche expiratoire 18 du circuit principal, ainsi qu'une valve d'échappement 10 permettant d'évacuer tout surplus gazeux éventuel et/ou toute surpression gazeuse éventuelle dans la branche expiratoire 18.

Par ailleurs, le ventilateur de l'invention comporte, de façon connue en soi, un soufflet 4 de ventilation mécanique incorporé dans une enceinte, ainsi qu'un ballon de ventilation manuelle 5, lesquels peuvent être sélectivement reliés fluidiquement au circuit principal CP pour l'alimenter en gaz sous pression, via un sélecteur 6 soufflet/ballon.

Des moyens de pilotage 3 comprenant, par exemple, au moins une carte électronique de contrôle et un ou plusieurs logiciel ou programmes informatiques embarqués, permettent de recueillir au moins une partie des informations ou signaux issus de tout ou partie des capteurs de l'appareil et de les traiter et/ou de réaliser tous les calculs nécessaires au suivi des concentrations en gaz et/ou à la commande des différents éléments de l'appareil.

En particulier, un capteur 11 de débit inspiratoire et un capteur 12 de débit expiratoire, agencés respectivement sur les branches inspiratoire 16 et expiratoire 18 du circuit principal (CP), mesurent les débits inspiratoires et respiratoires dans lesdites branches et transmettent les signaux de mesure ainsi obtenus aux moyens de pilotage 3 via des liaisons électriques adaptées. De cette manière les moyens de pilotage 3 sont aptes à commander le soufflet 4 et/ou l'ouverture de la valve d'échappement 10 et/ou l'entrée des gaz appropriés dans le bloc d'entrée 1 auxquels sont reliés lesdits moyens de pilotage 3, via des liaisons électriques dédiées, comme visible sur la figure 1.

Afm de pouvoir réaliser une mesure et un suivi efficace de la teneur en xénon du mélange gazeux, l'appareil de l'invention incorpore un module S6 d'analyse de gaz appelé "banc gaz" incluant un (ou plusieurs) capteur à fil(s) chaud(s) balayé par un flux gazeux dérivé. Le module S6 d'analyse de gaz est représenté de façon agrandie et détaillée sur la Figure 1 à l'extrémité de la flèche courbe.

Plus précisément, une partie du flux de gaz à base de xénon véhiculé par le circuit principal CP de gaz est prélevé, au niveau de la pièce 17 en Y, via une ligne de prélèvement S1 qui communique fluidiquement avec ledit le circuit principal CP.

La ligne S1 véhicule le gaz anesthésique jusqu'au module S6 en le faisant préalablement transiter par un piège à eau S2, où la vapeur d'eau qu'il contient est éliminée, avant d'être convoyé, via une ligne de transfert S3, jusqu'au module d'analyse de gaz S6.

Le module S6 d'analyse de gaz comprend, quant à lui, agencé sur le passage du flux de gaz :
- une pompe d'aspiration S6-A, par exemple du type de celle équipant les bancs gaz BGA4800 ou BGA4700 de la société ANDROS ou AION de la société ARTEMA, pour créer un débit d'aspiration de gaz anesthésique connu,
- un capteur à fil(s) chaud(s) S6-E, constitué dans l'exemple d'un fil de platine unique, parcouru par un courant électrique d'intensité (I) donnée, par exemple une intensité de 100 mA environ, avec mesure de la tension aux bornes dudit fil lorsque celui-ci est en contact avec le flux contenant le xénon, par exemple un capteur FIL CHAUD de la société TAEMA, permettant de mesurer la concentration en xénon
- une cellule infrarouge S6-B, du type par exemple de celle équipant les bancs gaz BGA4800 ou BGA4700 susmentionnés, permettant de mesurer les concentrations. instantanée et/ou moyenne et/ou inspirée et/ou expirée de dioxyde de CO₂, de N₂O, d'halogénés, d'éthanol ou de tout autre gaz mesurable par cette technologie infrarouge,
- une cellule paramagnétique à O₂ ou une pile chimique S6-C , du type par exemple de celle équipant les bancs gaz BGA4800 ou BGA4700 susmentionnés, pour mesurer les concentrations instantanées et/ou moyenne et/ou inspirée et/ou expirée de O₂,
- des moyens de commande S6-D à logiciel intégré sur une carte électronique de contrôle , du type par exemple de celle équipant les bancs gaz BGA4800 ou BGA4700 susmentionnés,
- des liaisons appropriées reliant la cellule infrarouge S6-B et la cellule à oxygène S6-C aux moyens de commande S6-D.

La sortie de la pompe d'aspiration S6-A du module S6 est reliée à la branche expiratoire du circuit principal, via une ligne de ré-injection S4, de manière à y renvoyer le gaz qui y a été prélevé par la ligne de prélèvement S1.

Par ailleurs, comme représenté, les signaux de mesure obtenus avec le capteur à fil(s) chaud(s) S6-E sont transmis aux moyens de commandes S6-D, via une liaison adaptée S6-F, lesdits moyens de commande S6-D étant eux-mêmes reliés, via une liaison S5 électrique adaptée, aux moyens de pilotage 3.

Les calculs notamment de concentrations en xénon du gaz anesthésique sont effectués par les moyens de commandes S6-D du module S6.

Ce module S6 d'analyse de gaz permet donc de réaliser toutes les mesures souhaitées sur le gaz aspiré par la ligne de prélèvement S1 à un débit continu.

Il est à noter que le capteur à fil(s) chaud(s) S6-E, bien que représenté en entrée du module S6 et en amont de la cellule S6-C, peut être aussi inséré ailleurs, en particulier en aval de la pompe d'aspiration S6-A et/ou en amont de ou sur la ligne de ré-injection S4, cette dernière étant connectée ou non au circuit principal.

Le capteur à fil(s) chaud(s) S6-E réalise, en temps réel, la mesure de la tension aux bornes du fil chaud générée par le gaz aspiré et la transmet par la liaison S6-F, avec un retard connu, plus ou moins court, de quelques dizaines voire quelques centaines de ms en fonction du débit d'aspiration réglé, au logiciel de contrôle S6-D de l'analyseur de gaz d'anesthésie pour que celui-ci en déduise notamment une mesure en temps réel de la teneur en xénon (Xe%), de la fraction inspirée en xénon (FiXe) ou de la fraction expirée en xénon (FeXe), voire même une concentration moyenne en xénon, comme expliqué dans WO-A-2007/068849.

Les Figures 1 à 4 proposent plusieurs modes de réalisation d'un appareil selon l'invention comprenant des moyens permettant d'effectuer une mesure plus précise de la concentration en xénon.

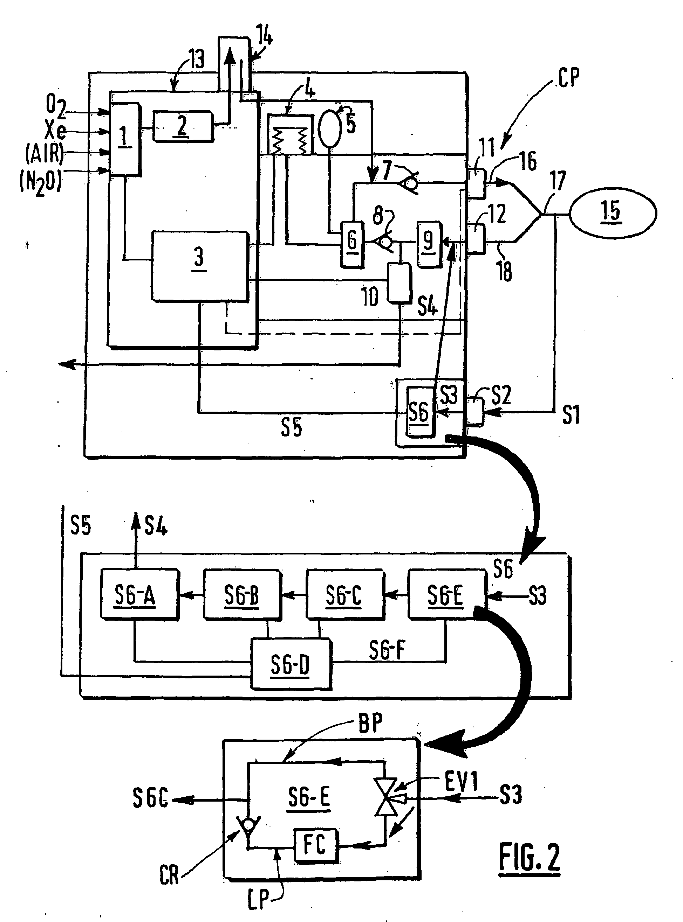

Ainsi, comme illustré sur les Figures 1 à 3, l'invention repose sur l'incorporation au niveau du capteur à fil(s) chaud(s) S6-E, d'une ou plusieurs électrovannes EV1, EV2 en amont et éventuellement en aval dudit capteur à fil(s) chaud(s) S6-E. En effet, cette ou ces deux électrovannes EV1, EV2 permettent :
- de contrôler l'acheminement de l'échantillon gazeux jusqu'au dispositif de mesure par prélèvement et/ou par aspiration de la pompe, par exemple du module S6,
- d'interrompre la circulation de l'échantillon gazeux à mesurer dans une partie du dispositif où se trouve un fil chaud FC en laissant s'écouler le débit de prélèvement par une ligne de fluide ou ligne de dérivation ou bypass BP qui se trouve en parallèle de la ligne principale LP sur laquelle est agencé le dispositif de mesure, c'est-à-dire le fil chaud FC, comme montré sur les Figures 1 et 2,
- au calculateur de réaliser la mesure de la concentration en xénon dans le gaz pendant un temps donné durant lequel l'échantillon à mesurer se trouve à débit nul puisque l'électrovanne EV1 est fermé. A l'issue de ce temps de mesure, l'échantillon gazeux dont le principe est basé sur l'échantillonneur bloqueur (comme expliqué ci-après), continue son chemin normal au travers du dispositif de mesure, c'est-à-dire de EV1 vers EV2 (sur la Figure 1 par exemple). Pendant le temps où la mesure est effectuée, l'échantillon gazeux ne doit pas être interrompu, il est véhiculé par la ligne de dérivation BP qui se trouve en parallèle avec le dispositif de mesure.

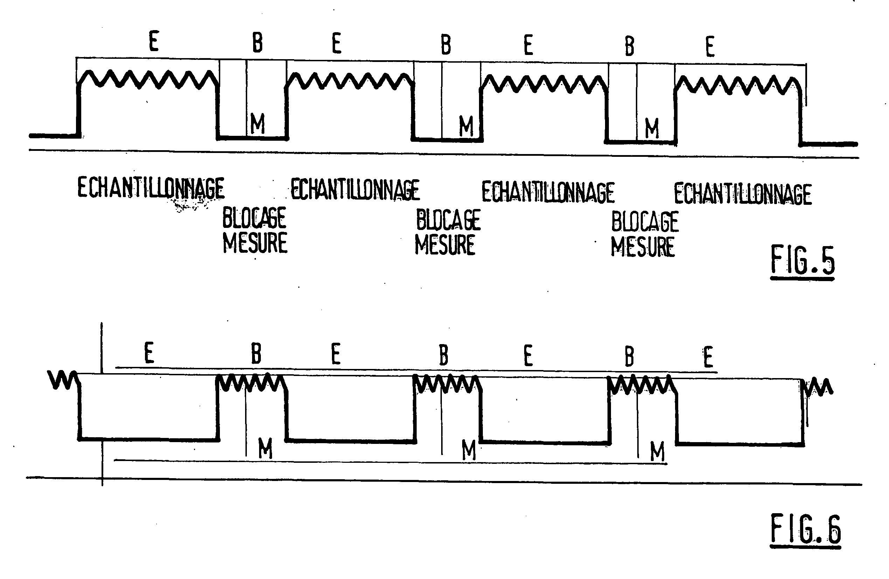

En fait, le principe de la mesure de xénon dans le mode de réalisation des Figures 1 et 2 est basé sur celui de l'échantillonneur bloqueur, comme illustré sur les chronogrammes de la Figure 5 qui représente le débit gazeux vu par le capteur à fil chaud FC utilisé pour réaliser la mesure de concentration en xénon, et de la Figure 6 qui illustre le débit gazeux passant dans la conduite de dérivation BP qui est agencée en parallèle de la chambre située sur la ligne principale LP et contenant le capteur à fil chaud FC utilisé pour réaliser la mesure de concentration en xénon.

En fait, pendant la phase dite « d'Echantillonnage » (E), le débit de prélèvement est orienté par l'électrovanne EV1, via la ligne principale LP, pour traverser la chambre où est agencé le fil chaud FC. Pendant cette phase, on mesure l'amplitude du signal électrique aux bornes du fil chaud pendant la phase d'échantillonnage pour vérifier que le débit passe et ainsi garantir que la prochaine mesure M sera cohérente.

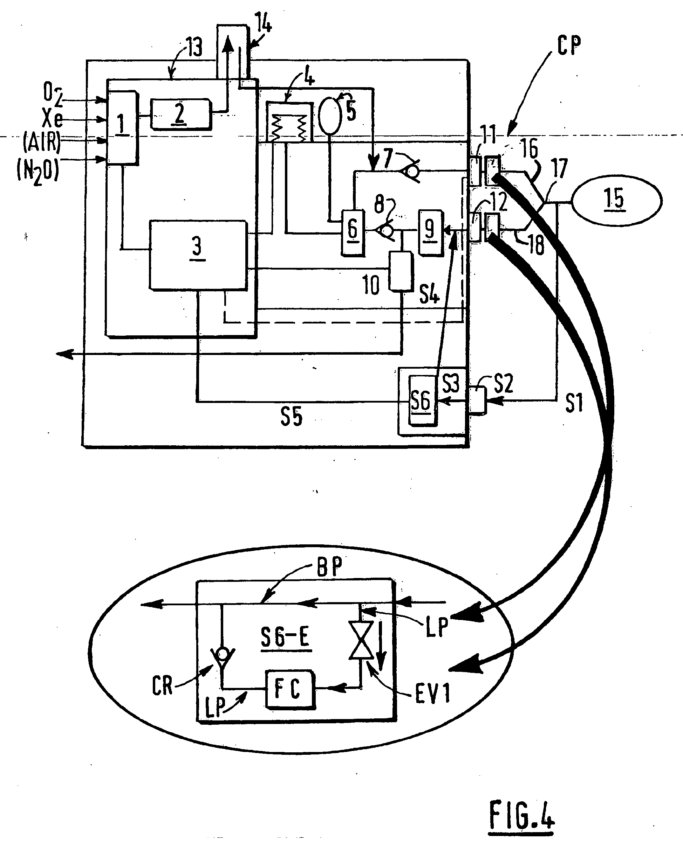

Pendant la phase dite « de Blocage » (B), le débit de prélèvement est orienté par l'électrovanne EV1 vers la conduite de bypass BP de telle sorte que le gaz à mesurer est emmagasiné dans la cellule FC que forme le capteur de débit isolé entre EV1 et EV2 (sur la figure 1) ou entre EV1 et CR (sur les figures 2 et 4) et se trouve à débit nul. Durant la seconde partie de la phase de blocage, on réalise la mesure de concentration de xénon comme détaillé dans WO-A-2007/068849 en déduisant de la mesure de tension aux bornes du fil chaud, la concentration du xénon en utilisant parmi le réseau de droites V = f(débit) ( Xe), la droite correspondant à un débit nul. On peut ainsi aussi calculer la concentration moyenne de xénon dans le gaz prélevé en moyennant les mesures.

Optionnellement, on peut aussi réaliser une synchronisation des phases d'échantillonnage et blocage, par exemple sur la mesure de CO₂ ou de mesure de la pression, de façon à ce que la phase d'Echantillonnage E soit synchronisée avec la phase d'insufflation et la phase de Blocage B avec la phase d'expiration du patient ; la mesure M correspondant alors à la fraction inspirée de xénon, ou de façon à ce que la phase d'Echantillonnage E soit synchronisée avec la phase d'expiration et la phase de Blocage B avec la phase d'insufflation ; la mesure M correspondant alors à la fraction expirée de xénon.

A noter que la différence majeure entre les Figures 1 et 2 réside en la présence, dans le mode de réalisation de la Figure 1, de deux électrovannes EV1, EV2 situées en amont et aval du fil chaud FC, alors que dans celui de la Figure 2, seule une électrovanne EV1 est agencée en amont du fil chaud et un clapet anti-retour CR a été agencé en aval dudit fil chaud FC, sur la ligne principale LP d'acheminement de gaz.

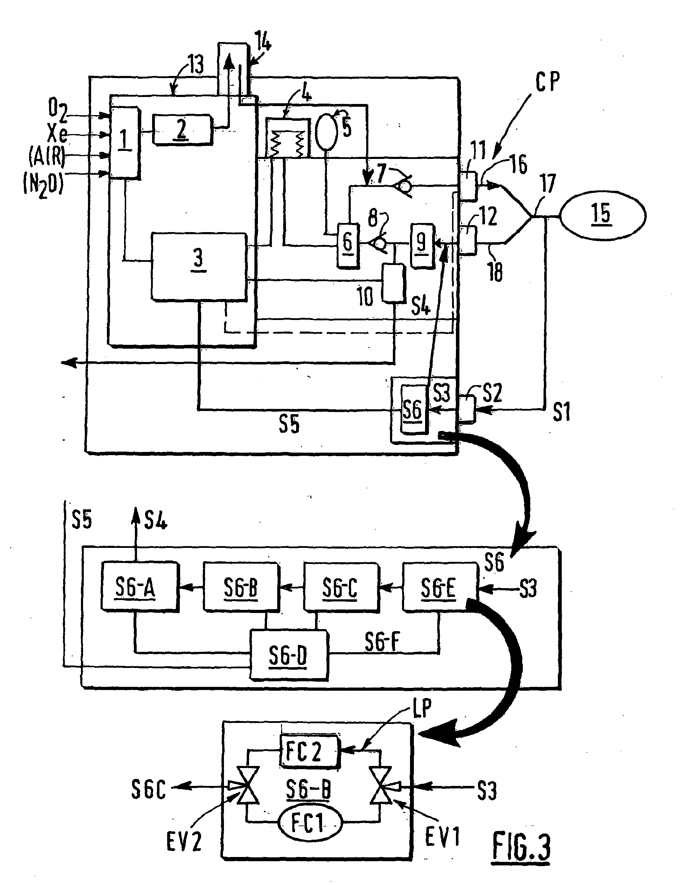

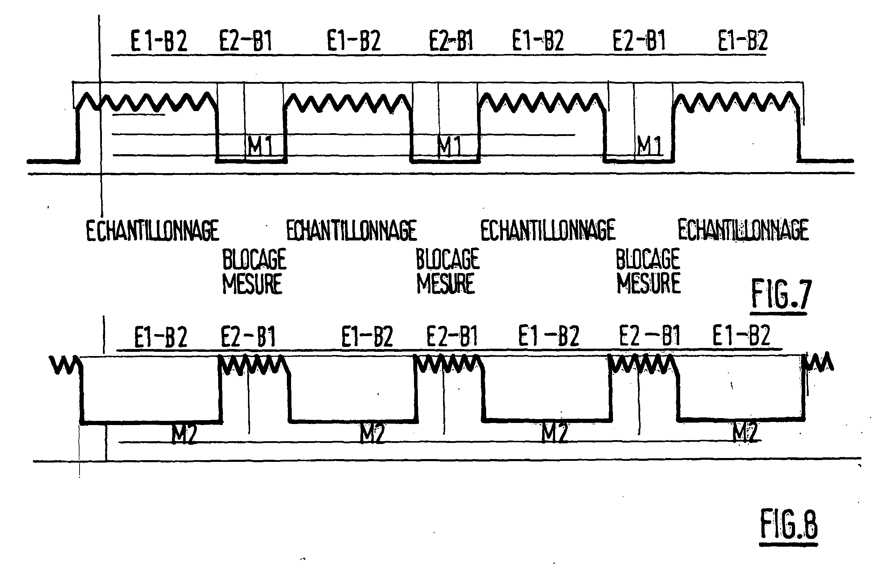

Par ailleurs, la figure 3 représente une autre variante du mode de réalisation de la figure 1 dans lequel la mesure est basée sur le principe d'un double échantillonneur-bloqueur (voir chronogrammes des Figures 7 et 8) permettant de mesurer la concentration de xénon en continu. Dans ce cas, on utilise deux capteurs à fils chauds pour mesurer la teneur en xénon, à savoir un premier capteur à fils chauds, FC1, et un deuxième capteur à fils chauds, FC2, deux capteurs bien distincts pouvant chacun comporter un ou plusieurs fils chauds.

Plus précisément, comme précédemment, la Figure 7 représente le débit vu par le capteur à fil chaud FC 1 utilisé pour réaliser la mesure de concentration en xénon, alors que la Figure 8 illustre le débit vu par le capteur à fil chaud FC2 utilisé pour réaliser la mesure de concentration en xénon.

Pendant la phase dite d'Echantillonnage 1 - Blocage 2 (E1-B2), le débit de prélèvement est orienté pour traverser le premier fil chaud FC1, alors que l'échantillon précédent est à débit nul dans la chambre contenant le deuxième fil chaud FC2. Pendant cette phase, on estime mesure l'amplitude du signal électrique aux bornes du premier fil chaud FC1 pendant la phase d'échantillonnage pour vérifier que le débit passe et ainsi garantir que la prochaine mesure (M1) sera cohérente. Durant cette phase, on effectue, en outre, la mesure (M2) de la concentration de xénon de l'échantillon bloqué dans la chambre contenant le deuxième fil chaud FC2 en utilisant parmi le réseau de droite V = f(débit) (Xe ) la droite correspondant au débit nul

Pendant la phase dite d'Echantillonnage 2 - Blocage 1 (E2-B1), le débit de prélèvement est orienté pour traverser le deuxième fil chaud FC2, alors que l'échantillon précédant est à débit nul dans la chambre contenant le fil chaud FC 1. Pendant cette phase, on mesure l'amplitude du signal électrique aux bornes du premier fil chaud FC2 pendant la phase d'échantillonnage pour vérifier que le débit passe et ainsi garantir que la prochaine mesure (M2) sera cohérente. Durant cette phase, on effectue, en outre, la mesure (M1) de la concentration de xénon de l'échantillon bloqué dans la chambre contenant le premier fil chaud FC1 en utilisant parmi le réseau de droite V = f(débit) (Xe), la droite correspondant au débit nul, comme expliqué ci-dessus.

On peut ainsi, si besoin est, calculer la concentration moyenne de xénon dans le gaz prélevé en moyennant les mesures M1 et M2.

Optionnellement, comme précédemment, on peut aussi réaliser une synchronisation des phases d'échantillonnage et blocage, par S6-D, par exemple sur les cycles de CO₂, détectés par S6-C, de façon à ce que sur un des capteurs à fil chaud (FC1 par exemple), la phase d'Echantillonnage soit synchronisée avec la phase d'insufflation et la phase de Blocage avec phase d'expiration, la mesure M1 correspondant alors à la fraction inspirée de xénon, et de façon à ce que sur l'autre capteur à fil chaud (FC2 par exemple), la phase d'Echantillonnage soit synchronisée avec la phase d'expiration et la phase de Blocage avec la phase d'insufflation, la mesure M2 correspondant alors à la fraction expirée de xénon.

En d'autres termes, la figure 1 utilise deux électrovannes 3/2 (3 vers 2) en amont EV1 et en aval EV2 du dispositif de mesure de la concentration en xénon comprenant le fil chaud FC. Ces électrovannes commutent simultanément pour faire passer l'échantillon gazeux soit à travers le dispositif de mesure de la concentration, soit à travers la conduite de dérivation BP pendant que le dispositif réalise la mesure de la concentration à débit nul.

Par contre, la figure 2 n'utilise qu'une seule électrovanne EV1 située en amont du fil chaud FC pour diriger l'échantillon gazeux soit à travers le dispositif de mesure de la concentration, soit à travers la conduite de dérivation BP. Dans ce cas, un clapet anti-retour CR est situé en aval du fil chaud FC pour éviter tout retour de gaz vers le fil chaud.

Le dispositif S6E se trouve en série avec les différents capteurs S6C, S6B, S6A qui composent le banc gaz ; il peut être placé au début ou à la fin de la chaîne, il est indépendant des autres capteurs.

La Figure 4 illustre un second mode de réalisation dans lequel un capteur à fil chaud S6-E similaire à celui de la Figure 2, voire à celui de la Figure 1, est positionné en série, en aval ou en amont, du capteur de débit inspiratoire 11 et ainsi permettre de mesurer la fraction inspirée de xénon en synchronisant la phase de blocage sur la phase d'expiration par détection de la disparition d'une pression positive d'insufflation ou par détection d'un débit d'expiration.

En outre, un autre capteur à fil chaud S6-E peut également être positionné de façon analogue en série avec le capteur de débit expiratoire 12 et ainsi permettre de mesurer la fraction expirée de xénon en synchronisant la phase de blocage sur la phase d'insufflation par détection d'une pression positive d'insufflation ou d'un débit d'insufflation.

Bien entendu, le (ou les) capteur(s) à fil(s) chaud(s) utilisé dans le cadre de l'invention peut comprendre un ou plusieurs fils composés de tout matériau conducteur de l'électricité approprié, notamment du platine.

Dans tous les cas, l'appareil de l'invention est utilisable en toute circonstance et en tout lieu, en particulier en bloc opératoire, durant les phases d'anesthésie au xénon, de manière à améliorer la sécurité des patients et s'inscrit dans le cadre des obligations de surveillance des gaz anesthésiants. Dans un tel gaz, le xénon gazeux est toujours mélangé avec de l'oxygène seul, de l'air ou alors avec de l'oxygène et éventuellement un ou plusieurs composés halogénés et/ou avec du protoxyde d'azote.

## Revendications

1. Appareil d'anesthésie ventilatoire d'un patient par administration d'un gaz contenant du xénon gazeux comprenant :
- un circuit principal de gaz (CP) en circuit ouvert ou fermé comportant une branche inspiratoire (16) pour acheminer un mélange gazeux contenant du xénon vers le patient et une branche expiratoire (18) pour véhiculer le mélange gazeux contenant du xénon expiré par le patient, et
- des moyens de détermination de concentration de xénon (S6, M1) conçus pour et aptes à déterminer la teneur en xénon gazeux dans au moins une partie du circuit principal (CP), lesdits moyens de détermination de concentration de xénon (S6 ; M1) comprenant au moins un capteur à fil(s) chaud(s) (S6-E) comportant au moins un fil conducteur (FC1) de l'électricité en contact direct avec au moins une partie du flux gazeux contenant le xénon, **caractérisé en ce que** :
- au moins un capteur à fil(s) chaud(s) (S6-E) comportant au moins un fil conducteur (FC1) est agencé sur une ligne principale (LP) d'acheminement de gaz comportant une ligne de dérivation (BP) se raccordant fluidiquement à ladite ligne principale (LP) d'acheminement de gaz en amont et en aval dudit au moins un fil conducteur (FC1), en considérant le sens de circulation du gaz dans la ligne principale (LP), et
- au moins une première électrovanne (EV1) est agencée au niveau du raccordement amont de la ligne de dérivation (BP) à ladite ligne principale (LP) de manière à diriger le flux gazeux contenant le xénon soit vers la ligne principale (LP) sur laquelle est agencé ledit au moins un fil conducteur (FC1), soit vers la ligne de dérivation (BP).

2. Appareil selon la revendication 1, **caractérisé en ce que** la ligne principale (LP) d'acheminement de gaz et la ligne de dérivation (BP) sont des ramifications d'une ligne de dérivation (S1, S3, S4) communiquant fluidiquement avec le circuit principal (CP) et **en ce qu'**au moins une première électrovanne (BV1) est agencée à l'intersection de ladite la ligne de dérivation (S1, S3, S4), de la ligne principale (LP) d'acheminement de gaz et la ligne de dérivation (BP), de préférence le raccordement de la ligne de dérivation (S1, S3, S4) au circuit principal (CP) se fait sur la branche inspiratoire (16) et/ou sur la branche expiratoire (18) et/ou en un site localisé à proximité immédiatement de la bouche du patient, de préférence encore au niveau d'un site de raccordement (17) entre la branche inspiratoire (16) et la branche expiratoire (18) dudit circuit principal (CP), par exemple au niveau d'une pièce de raccordement en Y ou d'un filtre bactériologique agencé sur le circuit principal (CP).

3. Appareil selon la revendication 1, **caractérisé en ce qu'**au moins un capteur à fil(s) chaud(s) est agencé directement sur la branche inspiratoire (16) ou expiratoire (18) du circuit principal (CP), et **en ce que** la ligne principale (LP) d'acheminement de gaz et la ligne de dérivation (BP) sont des ramifications de la branche inspiratoire (16) ou expiratoire (18) du circuit principal (CP), et **en ce qu'**au moins une première électrovanne (EV1) est agencée à l'intersection de la branche inspiratoire (16) ou expiratoire (18), de la ligne principale (LP) et la ligne de dérivation (BP).

4. Appareil selon l'une quelconque des revendications 1 à 3, **caractérisé en ce qu'**au moins une deuxième électrovanne (EV2) est agencée au niveau du raccordement aval de la ligne de dérivation (BP) à ladite ligne principale (LP).

5. Appareil selon l'une quelconque des revendications 1 à 3, **caractérisé en ce qu'**au moins un clapet anti-retour (CR) est agencé sur la ligne principale (LP) d'acheminement de gaz, entre au moins un fil conducteur (FC1) et le raccordement aval de la ligne de dérivation (BP) à ladite ligne principale (LP).

6. Appareil selon l'une quelconque des revendications 1 à 4, **caractérisé en ce qu'**au moins un deuxième capteur à fil conducteur (FC2) est agencé sur la ligne de dérivation (BP) qui mesure la concentration en xénon par exemple pendant la phase expiratoire alors qu'au moins un fil conducteur (FC1) mesure la concentration pendant la phase inspiratoire ou inversement.

7. Appareil selon la revendication 3, **caractérisé en ce qu'**au moins un capteur à fil(s) chaud(s) est agencé sur la branche inspiratoire (16), en amont ou en aval d'un capteur de débit inspiratoire (11) pour permettre de mesurer la fraction inspirée de xénon.

8. Appareil selon la revendication 3, **caractérisé en ce qu'**au moins un capteur à fil(s) chaud(s) est agencé sur la branche expiratoire (18), en amont ou en aval d'un capteur de débit expiratoire (12) pour permettre de mesurer la fraction expirée de xénon.

9. Appareil selon l'une quelconque des revendications 1 à 8, **caractérisé en ce que** les électrovannes (EV1, EV2) sont commandées par des moyens de pilotage (3) ou par une interface indépendante.

10. Appareil selon l'une quelconque des revendications précédentes, **caractérisé en ce qu'**il comprend :
- des moyens d'alimentation (1, 2) en xénon gazeux reliés au circuit principal pour alimenter la branche inspiratoire (16) du circuit principal (CP) avec un gaz contenant du xénon, et
- des moyens de calcul (3 ; S6-D) coopérant avec au moins un capteur à fil(s) chaud(s) (S6-E) de manière à déterminer la concentration de xénon dans ledit flux gazeux,
- des moyens de génération de courant électrique apte à et conçus pour générer un courant un électrique dans au moins un fil chaud dudit au moins un capteur à fil(s) chaud(s) (S6-E),
- des moyens de mesure de tension aptes à mesurer au moins une valeur de tension aux bornes d'au moins un fil chaud dudit au moins un capteur à fil chaud (S6-E) ou aux bornes d'au moins une résistance agencée en série avec au moins un fil chaud dudit au moins un capteur à fil chaud (S6-E), lorsque ledit au moins un fil chaud est en contact avec le flux gazeux et est parcouru par un courant électrique d'intensité non nulle,
- et **en ce que** les moyens de calcul (3 ; S6-D) coopèrent avec les moyens de mesure de tension de manière à déterminer, à partir de la mesure de tension effectuée par lesdits moyens de mesure de tension, la concentration de xénon dans ledit flux.

## Patentansprüche

1. Anästhesiegerät zur Beatmung eines Patienten durch Verabreichung eines Gases, das gasförmiges Xenon enthält, umfassend:
- einen Hauptgaskreislauf (CP) im offenen oder geschlossenen Kreislauf, der einen inspiratorischen Zweig (16) umfasst, um ein xenonhaltiges Gasgemisch zum Patienten zu befördern, und einen exspiratorischen Zweig (18) umfasst, um das vom Patienten ausgeatmete, xenonhaltige Gasgemisch zu transportieren, und
- Mittel zum Bestimmen der Xenonkonzentration (S6, M1), die ausgelegt und geeignet sind, um den Gehalt an gasförmigem Xenon in mindestens einem Teil des Hauptkreislaufs (CP) zu bestimmen, wobei die Mittel zum Bestimmen der Xenonkonzentration (S6; M1) mindestens einen Sensor mit einem oder mehreren Hitzdrähten (S6-E) umfassen, der mindestens einen Stromleitungsdraht (FC1) in unmittelbarem Kontakt mit mindestens einem Teil des xenonhaltigen Gasstroms umfasst,
**dadurch gekennzeichnet, dass**
- mindestens ein Sensor mit einem oder mehreren Hitzdrähten (S6-E), der mindestens einen Leitungsdraht (FC1) umfasst, auf einer Hauptgasbeförderungsleitung (LP) angeordnet ist, die eine Umgehungsleitung (BP) umfasst, die fluidtechnisch an die Hauptgasbeförderungsleitung (LP) mit Bezug auf die Umlaufrichtung des Gases in der Hauptleitung (LP) gesehen oberhalb und unterhalb des mindestens einen Leitungsdrahts (FC1) angeschlossen wird, und
- mindestens ein erstes Elektroventil (EV1) am oberen Anschluss der Umgehungsleitung (BP) an die Hauptleitung (LP) derart angeordnet ist, dass es den xenonhaltigen Gasstrom entweder auf die Hauptleitung (LP), auf welcher der mindestens eine Leitungsdraht (FC1) angeordnet ist, oder auf die Umgehungsleitung (BP) richtet.

2. Gerät nach Anspruch 1, **dadurch gekennzeichnet, dass** die Hauptgasbeförderungsleitung (LP) und die Umgehungsleitung (BP) Verzweigungen einer Umgehungsleitung (S1, S3, S4) sind, die fluidtechnisch mit dem Hauptkreislauf (CP) in Verbindung steht, und dass mindestens ein erstes Elektroventil (EV1) am Schnittpunkt der Umgehungsleitung (S1, S3, S4), der Hauptgasbeförderungsleitung (LP) und der Umgehungsleitung (BP) angeordnet ist, der Anschluss der Umgehungsleitung (S1, S3, S4) an den Hauptkreislauf (CP) auf dem inspiratorischen Zweig (16) und/oder auf dem exspiratorischen Zweig (18) und/oder an einer Stelle, die sich in unmittelbarer Nähe des Mundes des Patienten befindet, bevorzugt noch an einer Anschlussstelle (17) zwischen dem inspiratorischen Zweig (16) und dem exspiratorischen Zweig (18) des Hauptkreislaufs (CP), beispielsweise an einem Y-förmigen Anschlussstück oder einem bakteriologischen Filter, der am Hauptkreislauf (CP) angeordnet ist, erfolgt.

3. Gerät nach Anspruch 1, **dadurch gekennzeichnet, dass** mindestens ein Sensor mit einem oder mehreren Hitzdrähten unmittelbar am inspiratorischen Zweig (16) oder am exspiratorischen Zweig (18) des Hauptkreislaufs (CP) angeordnet ist, und dass die Hauptgasbeförderungsleitung (LP) und die Umgehungsleitung (BP) Verzweigungen des inspiratorischen Zweigs (16) oder des exspiratorischen Zweigs (18) des Hauptkreislaufs (CP) sind, und dass mindestens ein erstes Elektroventil (EV1) am Schnittpunkt des inspiratorischen Zweigs (16) oder des exspiratorischen Zweigs (18) der Hauptleitung (LP) und der Umgehungsleitung (BP) angeordnet ist.

4. Gerät nach einem der Ansprüche 1 bis 3, **dadurch gekennzeichnet, dass** mindestens ein zweites Elektroventil (EV2) am unteren Anschluss der Umgehungsleitung (BP) an der Hauptleitung (LP) angeordnet ist.

5. Gerät nach einem der Ansprüche 1 bis 3, **dadurch gekennzeichnet, dass** mindestens ein Rückschlagventil (CR) an der Hauptgasbeförderungsleitung (LP) zwischen mindestens einem Leitungsdraht (FC1) und dem unteren Anschluss der Umgehungsleitung (BP) an der Hauptleitung (LP) angeordnet ist.

6. Gerät nach einem der Ansprüche 1 bis 4, **dadurch gekennzeichnet, dass** mindestens ein zweiter Sensor mit einem Leitungsdraht (FC2) auf der Umgehungsleitung (BP) angeordnet ist und die Xenonkonzentration beispielsweise während der exspiratorischen Phase misst, während mindestens ein Leitungsdraht (FC1) die Konzentration während der inspiratorischen Phase misst, oder umgekehrt.

7. Gerät nach Anspruch 3, **dadurch gekennzeichnet, dass** mindestens ein Sensor mit einem oder mehreren Hitzdrähten auf dem inspiratorischen Zweig (16) oberhalb oder unterhalb eines inspiratorischen Flowsensors (11) angeordnet ist, um die Messung des eingeatmeten Xenonanteils zu ermöglichen.

8. Gerät nach Anspruch 3, **dadurch gekennzeichnet, dass** mindestens ein Sensor mit einem oder mehreren Hitzdrähten auf dem exspiratorischen Zweig (18) oberhalb oder unterhalb eines exspiratorischen Flowsensors (12) angeordnet ist, um die Messung des ausgeatmeten Xenonanteils zu ermöglichen.

9. Gerät nach einem der Ansprüche 1 bis 8, **dadurch gekennzeichnet, dass** die Elektroventile (EV1, EV2) durch Steuerungsmittel (3) oder über eine unabhängige Schnittstelle gesteuert werden.

10. Gerät nach einem der vorhergehenden Ansprüche, **dadurch gekennzeichnet, dass** es Folgendes umfasst:
- Mittel (1, 2) zur Versorgung mit gasförmigem Xenon, die mit dem Hauptkreislauf verbunden sind, um den inspiratorischen Zweig (16) des Hauptkreislaufs (CP) mit einem xenonhaltigen Gas zu versorgen, und
- Rechenmittel (3; S6-D), die mit mindestens einem Sensor mit einem oder mehreren Hitzdrähten (S6-E) derart zusammenwirken, dass sie die Xenonkonzentration in dem Gasstrom bestimmen,
- Mittel zum Generieren von elektrischem Strom, die dazu geeignet und ausgelegt sind, um einen elektrischen Strom in mindestens einem Hitzdraht des mindestens einen Sensors mit einem oder mehreren Hitzdrähten (S6-E) zu generieren,
- Spannungsmessmittel, die dazu geeignet sind, um mindestens einen Spannungswert an den Klemmen mindestens eines Hitzdrahts des mindestens einen Sensors mit einem oder mehreren Hitzdrähten (S6E) oder an den Klemmen mindestens eines Widerstandes, der mit mindestens einem Hitzdraht des mindestens einen Hitzdrahtsensors (S6-E) in Reihe geschaltet ist, zu messen, wenn der mindestens eine Hitzdraht mit dem Gasstrom in Kontakt steht und ein elektrischer Strom einer Stärke ungleich Null durch diesen fließt,
- und dass die Rechenmittel (3; S6-D) mit den Spannungsmessmitteln derart zusammenwirken, dass sie aus der Spannungsmessung, die von den Spannungsmessmitteln ausgeführt wird, die Xenonkonzentration in dem Strom bestimmen.

## Claims

1. Apparatus for ventilatory anaesthesia of a patient by administration of a gas containing gaseous xenon, comprising:
- a main gas circuit (CP) in the form of an open or closed circuit having an inhalation branch (16) for supplying a gaseous mixture containing xenon to the patient and an exhalation branch (18) for conveying the gaseous mixture containing xenon exhaled by the patient, and
- means (S6, M1) for determining the concentration of xenon which are designed and able to determine the gaseous xenon content in at least part of the main circuit (CP), said means (S6, M1) for determining the concentration of xenon comprising at least one hot-wire sensor (S6-E) having at least one electrically conductive wire (FC1) in direct contact with at least part of the gaseous flow containing the xenon,
**characterised in that**:
- at least one hot-wire sensor (S6-E) having at least one conductive wire (FC1) is arranged on a gas-supply main line (LP) having a bypass line (BP) in fluid communication with said gas-supply main line (LP) upstream and downstream from said at least one conductive wire (FC1), as seen in the direction of circulation of the gas in the main line (LP), and
- at least a first solenoid valve (EV1) is arranged in the region of the upstream connection of the bypass line (BP) to said main line (LP) in such a way as to direct the gaseous flow containing the xenon either to the main line (LP), on which said at least one conductive wire (FC1) is arranged, or to the bypass line (BP).

2. Apparatus according to claim 1, **characterised in that** the gas-supply main line (LP) and the bypass line (BP) are ramifications of a bypass line (S1, S3, S4) in fluid communication with the main circuit (CP), and **in that** at least a first solenoid valve (EV1) is arranged at the intersection of said bypass line (S1, S3, S4), the gas-supply main line (LP) and the bypass line (BP), the connection of the bypass line (S1, S3, S4) to the main circuit (CP) is preferably made on the inhalation branch (16) and/or on the exhalation branch (18) and/or at a site located in immediate proximity to the patient's mouth, more preferably in the region of a connection site (17) between the inhalation branch (16) and the exhalation branch (18) of said main circuit (CP), for example in the region of a Y-shaped connection piece or of a bacteriological filter arranged on the main circuit (CP).

3. Apparatus according to claim 1, **characterised in that** at least one hot-wire sensor is arranged directly on the inhalation (16) or exhalation (18) branch of the main circuit (CP), and **in that** the gas-supply main line (LP) and the bypass line (BP) are ramifications of the inhalation (16) or exhalation (18) branch of the main circuit (CP), and **in that** at least a first solenoid valve (EV1) is arranged at the intersection of the inhalation (16) or exhalation (18) branch, the main line (LP) and the bypass line (BP).

4. Apparatus according to any of claims 1 to 3, **characterised in that** at least a second solenoid valve (EV2) is arranged in the region of the downstream connection of the bypass line (BP) to said main line (LP).

5. Apparatus according to any of claims 1 to 3, **characterised in that** at least one non-return valve (CR) is arranged on the gas-supply main line (LP), between at least one conductive wire (FC1) and the downstream connection of the bypass line (BP) to said main line (LP).

6. Apparatus according to any of claims 1 to 4, **characterised in that** at least a second conductive wire sensor (FC2) is arranged on the bypass line (BP) and measures the xenon concentration for example during the exhalation phase while at least one conductive wire (FC1) measures the concentration during the inhalation phase, or vice versa.

7. Apparatus according to claim 3, **characterised in that** at least one hot-wire sensor is arranged on the inhalation branch (16), upstream or downstream from an inhalation flow rate sensor (11), so as to permit measurement of the inhaled fraction of xenon.

8. Apparatus according to claim 3, **characterised in that** at least one hot-wire sensor is arranged on the exhalation branch (18), upstream or downstream from an exhalation flow rate sensor (12), so as to permit measurement of the exhaled fraction of xenon.

9. Apparatus according to any of claims 1 to 8, **characterised in that** the solenoid valves (EV1, EV2) are controlled by control means (3) or by an independent interface.

10. Apparatus according to any of the preceding claims, **characterised in that** it comprises:
- means (1, 2) for supplying gaseous xenon which are connected to the main circuit in order to supply the inhalation branch (16) of the main circuit (CP) with a gas containing xenon, and
- calculating means (3; S6-D) cooperating with at least one hot-wire sensor (S6-E) in such a way as to determine the concentration of xenon in said gaseous flow,
- means for generating electric current which are able and designed to generate an electric current in at least one hot wire of said at least one hot-wire sensor (S6-E),
- voltage-measuring means which are able to measure at least one voltage value at the terminals of at least one hot wire of said at least one hot-wire sensor (S6-E) or at the terminals of at least one resistor arranged in series with at least one hot wire of said at least one hot-wire sensor (S6-E), when said at least one hot wire is in contact with the gaseous flow and is traversed by an electric current of non-zero intensity,
- and **in that** the calculating means (3; S6-D) cooperate with the voltage-measuring means in such a way as to determine, from the voltage measurement carried out by said voltage-measuring means, the concentration of xenon in said flow.
